# EUROPEAN PATENT APPLICATION

(11) **EP 4 591 871 A1**
(43) Date of publication of application: **30.07.2025**
(21) Application number: 23860211.4
(22) Date of filing: 25.08.2023
(51) Int. Cl.: A61K 35/741, A61P 3/04, A61P 43/00

(54) **ANTI-OBESITY AGENT HAVING INTESTINAL BACTERIA AS ACTIVE INGREDIENT, AND METHOD FOR MANUFACTURING SAME**

(30) Priority: 29.08.2022 JP 2022136372; 29.08.2022 JP 2022136373
(71) Applicant: Kabushiki Kaisha Yakult Honsha, Tokyo, 105-8660 (JP)
(72) Inventor: IBUKI Tatsuya, Tokyo 105-8660 (JP); YAMAZAKI Ryuta, Tokyo 105-8660 (JP); HAIBARA Hirotaka, Tokyo 105-8660 (JP)
(74) Representative: Müller-Boré & Partner Patentanwälte PartG mbB
(86) International application number: PCT/JP2023/030711
(87) International publication number: WO 2024/048449

(57) **Abstract**

A novel anti-obesity agent and a method for producing the same are provided.

An anti-obesity agent comprising Alistipes bacteria as an active ingredient.

## Description

### Technical Field

The present invention relates to an anti-obesity agent containing enterobacteria as an active ingredient, and a method for producing the same.

### Background Art

Obesity refers to a disease state in which the body is abnormally obese due to excessive lipid deposition, and is one of large patient-scale diseases worldwide. In addition, obesity has a risk of promoting diabetes, hyperlipidemia, hypertension, and the like, and is a disease to be treated immediately.

However, it is difficult to develop therapeutic agents for obesity (anti-obesity agents) from the viewpoint of insufficient effects, side effects, and the like, and there are few anti-obesity agents currently approved in Japan (Non Patent Literature 1).

On the other hand, with the development of DNA analysis and the establishment of enterobacteriology in recent years, it has been revealed that a change in the intestinal environment is closely involved with the maintenance of host biological homeostasis. For example, Akkermansia muciniphila (referred to as "AM") is enterobacteria using mucin as a single nutrient source, and has been reported to be associated with obesity (Non Patent Literature 2).

### Citation List

### Non Patent Literature

Non Patent Literature 1: Journal of Japan Society, Vol. 84, p. 1290 to 1294, 1995
Non Patent Literature 2: Gut, Vol. 65, p. 426 to 436, 2016

### Summary of Invention

### Technical Problem

However, regarding enterobacteria other than AM, knowledge related to obesity has not yet been obtained, and there remains room for research and development.

Therefore, an object of the present invention is to newly identify enterobacteria having an anti-obesity action, and to provide a novel anti-obesity agent based on the action and a method for producing the same.

### Solution to Problem

As a result of intensive studies, the present inventors have unexpectedly found that Alistipes bacteria are suitable for use as an anti-obesity agent. That is, the characteristics of the anti-obesity agent of the present invention are as follows.

[1] An anti-obesity agent containing Alistipes bacteria or membrane vesicles (MVs) thereof as an active ingredient.
[2] The anti-obesity agent according to [1], wherein the anti-obesity agent is a differentiation suppressant for adipocytes, a lipid accumulation suppressant, or an agent for reducing accumulated lipid.
[3] The anti-obesity agent according to [1] or [2], wherein the bacteria is Alistipes indistinctus or Alistipes finegoldii.
[4] A method for producing an anti-obesity agent containing Alistipes bacteria or membrane vesicles (MVs) thereof as an active ingredient, the method comprising: a step a of culturing Alistipes bacteria; and
   a step b of acquiring the bacteria from the culture solution cultured in the step a.
[5] The method for producing the anti-obesity agent according to [4], wherein the step b further includes a step of centrifuging and filtering the culture solution to obtain the MVs.

### Advantageous Effects of Invention

According to the present invention, it is possible to provide an anti-obesity agent containing Alistipes bacteria as an active ingredient, and a method for producing the same.

### Brief Description of Drawings

Fig. 1 is a view showing a process of differentiation of an adipocyte.
Fig. 2 is a view schematically illustrating membrane vesicles.
Fig. 3 is a photograph showing membrane vesicles of Alistipes indistinctus (AI) in the upper part, and membrane vesicles of AM in the lower part.
Fig. 4A is a photograph showing that heated killed bacteria (HKB) of AM and AI suppress differentiation of adipocytes.
Fig. 4B is a bar graph showing that HKB of AM and AI suppresses differentiation of adipocytes.
Fig. 5A is a photograph showing that membrane vesicles (MVs) of AM and AI suppress differentiation of adipocytes.
Fig. 5B is a bar graph showing that MVs of AM and AI suppress differentiation of adipocytes.
Fig. 6 is views showing AM and AI suppress protein expression (Figs. 6(A) and 6(B)) and mRNA expression (Fig. 6(C)) of factors related to differentiation of adipocytes.
Fig. 7 is a view showing that AM and AI suppress secretion of adiponectin.
Fig. 8A is a photograph showing that HKB of AI and Alistipes finegoldii (AF) suppresses differentiation of adipocytes.
Fig. 8B is a bar graph showing that HKB of AI and AF suppresses differentiation of adipocytes.
Fig. 9A is a photograph showing that MVs of AI and AF suppress differentiation of adipocytes.
Fig. 9B is a bar graph showing that MVs of AI and AF suppress differentiation of adipocytes.
Fig. 10 is views showing that AI and AF suppress protein expression (Fig. 10(A)) and mRNA expression (Fig. 10(B)) of factors related to differentiation of adipocytes.
Fig. 11A is a photograph showing that AI and AF suppress accumulation of lipid oil droplets in adipocytes.
Fig. 11B is a bar graph showing that AI and AF suppress accumulation of lipid oil droplets in adipocytes.
Fig. 12A is a photograph showing that AI and AF reduce lipid oil droplets in adipocytes.
Fig. 12B is a bar graph showing that AI and AF reduce lipid oil droplets in adipocytes.
Fig. 13 is a table describing primers for detecting 16S rRNA of each bacterial cell.
Fig. 14A is a diagram showing detection amounts of MVs of AI and AM in feces.
Fig. 14B is a chart showing detection amounts of MVs of AI and AM in blood.
Fig. 15A is a chart showing a detection amount of MVs of AI in each tissue.
Fig. 15B is a chart showing detection amounts of MVs of AM in each tissue.

### Description of Embodiments

Hereinafter, an anti-obesity agent containing the enterobacteria of the present invention as an active ingredient is described in detail, but the description of the components described below is an example as an embodiment of the present invention, and is not limited to these contents.

The present inventors have unexpectedly discovered that Alistipes bacteria are the active ingredient of an anti-obesity agent. When used as the anti-obesity agent, for example, membrane vesicles (referred to as "MVs") extracted from bacteria or dead bacteria such as heated killed bacteria ("HKB") of bacteria may be used as the active ingredient. From the viewpoint of migration to tissues and the like, the form of membrane vesicles (MVs) is more preferable.

Also, Alistipes bacteria are, for example, Alistipes indistinctus (referred to as "AI") or Alistipes finegoldii (referred to as "AF").

In addition, the present inventors have unexpectedly found that the bacteria act to have an action of suppressing differentiation from precursor cells to adipocytes, an action of suppressing lipid accumulation in adipocytes, and an action of reducing accumulated lipid in enlarged adipocytes. This is described in more detail below.

In the present invention, the anti-obesity agent may be a pharmaceutical composition. In the case of formulation of a pharmaceutical composition, an acceptable additive may be used in combination. Examples of the additive include an excipient, a stabilizer, an antiseptic, a wetting agent, an emulsifier, a lubricant, a sweetener, a coloring agent, a flavoring agent, a buffering agent, an antioxidant, and a pH adjusting agent.

The anti-obesity agent contains Alistipes bacteria as the active ingredient, but may contain components other than the bacteria to such an extent that the effect of the present invention is not hindered. Examples of the component other than bacteria include medium components, solvents such as water, carbohydrates, proteins, lipids, vitamins, minerals, biologically essential trace metals (manganese sulfate, zinc sulfate, magnesium chloride, potassium carbonate, etc.), bacteria and probiotics other than Alistipes, and pharmaceutically acceptable carriers.

When the anti-obesity agent is an oral preparation, the anti-obesity agent can be in the form of a solid formulation such as a tablet, a powder, a fine granule, a granule, a capsule, a pill, or a sustained release, or a liquid formulation such as a solution, a suspension, or an emulsion.

The ingest of the anti-obesity agent of the present invention is not particularly limited as long as the effect of the present invention is exhibited. In addition, it can be appropriately adjusted according to the age, health condition, weight, and the like of the ingest person.

For example, it is desirable to ingest 1 x 10² or more, more preferably 1 x 10⁶ or more Alistipes bacteria as the active ingredient per day as a lower limit. In addition, it is desirable to ingest 1x10¹⁵ or less, more preferably 1x10¹¹ or less per day as an upper limit.

For example, it is desirable to ingest 0.1 µg or more, more preferably 1.0 µg or more of membrane vesicles (MVs) of Alistipes bacteria as the active ingredient per day as a lower limit. In addition, it is desirable to ingest 30 g or less, more preferably 15 g or less per day as an upper limit.

When the ingest per day is less than the lower limit value, the effect as the anti-obesity agent may be weakened, and when the ingest per day is more than the upper limit value, the effect as the anti-obesity agent may reach a certain level, and the effect per ingest may not exhibit.

It is desirable that the anti-obesity agent of the present invention is continuously ingested over a long period of time until the obesity is ideally eliminated. In order to better exhibit the effect, as a lower limit, for example, it is preferable to continuously ingest for four weeks or more, and it is more preferable to continuously ingest for several months to several years or more. When the period is less than the lower limit, the effect as the anti-obesity agent may be weakened.

When the anti-obesity agent is continuously ingested for a long period of time, there may be a case where the user neglects to ingest the anti-obesity agent due to unavoidable circumstances such as travel and work. In such a case, the frequency of ingest may be appropriately adjusted, for example, the anti-obesity agent is ingested 1 to 3 times a week, or the anti-obesity agent is ingested 2 to 3 times a day after several days apart.

The subject for ingesting the anti-obesity agent of the present invention is not particularly limited, and examples thereof include a person suffering from obesity, diabetes, hyperlipidemia, hypertension, lifestyle diseases, or the like, or a person who needs to prevent these diseases. The anti-obesity agent has not only an effect of treating the disease but also an effect of alleviating or ameliorating the deterioration of symptoms and an effect of preventing the disease.

Alistipes as the active ingredient of the anti-obesity agent is a kind of human enterobacteria belonging to the phylum Bacteroides. Alistipes bacteria of the present invention may include Alistipes ihumii, Alistipes inops Shkoporov, Alistipes onderdonkii, Alistipes putredinis, Alistipes shahii, Alistipes timonensis, Alistipes senegalensis, Alistipes obesi, Alistipes megaguti, Alistipes provecensis, Alistipes disper, Alistipes communis, Alistipes massiliensis, and the like, in addition to the above AI and AF.

Heated killed bacteria (referred to as "HKB") are obtained by subjecting live bacterial cells to a heat treatment for a predetermined time to kill bacteria. The killed bacteria may be subjected to, for example, a treatment such as concentration/dilution, freezing, drying, and powderization in addition to the heat treatment. The heat treatment is not particularly limited as long as the effect of the present invention is exhibited, and is performed under conditions usually used for sterilizing viable bacterial cells. The heat treatment is an example of a method for killing bacteria, and the method for killing bacteria is not limited to the heat treatment.

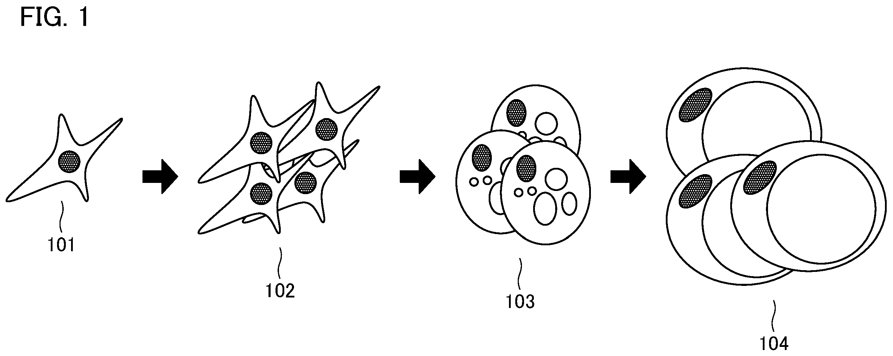

Differentiation of an adipocyte is described with reference to Fig. 1. The adipocytes include small adipocytes 103 and enlarged adipocytes 104 in which the small adipocytes 103 excessively store lipid droplets due to excessive ingest of calories or lack of exercise, and the size is increased. All the adipocytes are generated by differentiation of precursor cells 102 derived from mesodermal stem cells 101.

When the adipose tissue volume increases due to an increase in the number and size of adipocytes, symptoms of obesity appear. In addition, the enlarged adipocytes 104 produce bad adipocytokines to cause insulin resistance in the body, and as a result, diabetes, hyperlipidemia, hypertension, and the like are promoted.

Therefore, when a specific substance has an action of suppressing the process in which the precursor cells 102 are differentiated into the small adipocytes 103, suppressing the process in which the small adipocytes 103 excessively accumulate lipid droplets to form the enlarged adipocytes 104, or reducing lipid droplets already accumulated by the enlarged adipocytes 104, the substance can be regarded as the active ingredient of the anti-obesity agent.

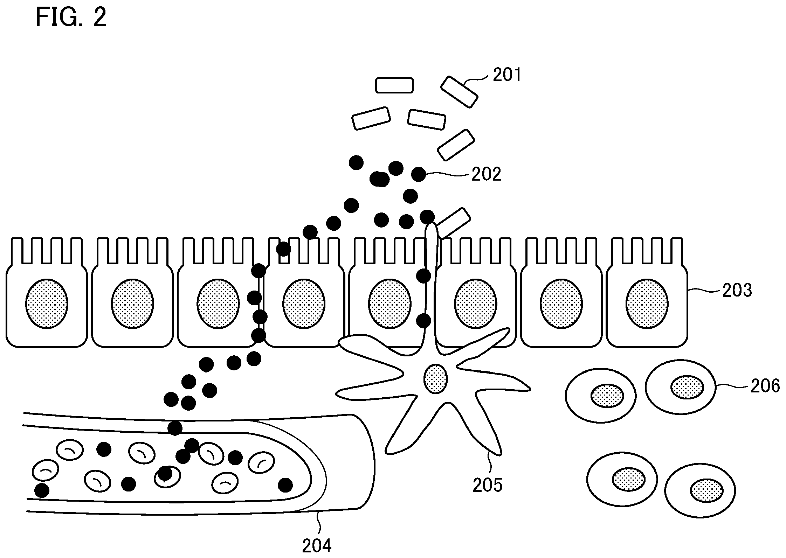

The membrane vesicles (MVs) is described with reference to Fig. 2. MV202 is a vesicle produced by live enterobacteria 201. Note that MV202 may be artificially produced as long as it exhibits the effect of MV disclosed in the present invention. When made from enterobacteria 201, MV202 is formed from the cell membrane of enterobacteria 201. Since the enterobacteria 201 generally have a size of about 1 µm, the enterobacteria themselves do not pass through the intercellular space of the intestinal epithelium 203, and the membrane component of MV202 or MV202 passes through the intercellular space of the intestinal epithelium 203, thereby acting on the precursor cells 102 of adipocytes (present in a distant place), the small adipocytes 103, the enlarged adipocytes 104, and the like via blood vessels 204 and the like. The enterobacteria 201 also function in intestinal homeostasis, such as acting on the T cells 206 and the like via the dendritic cell 205.

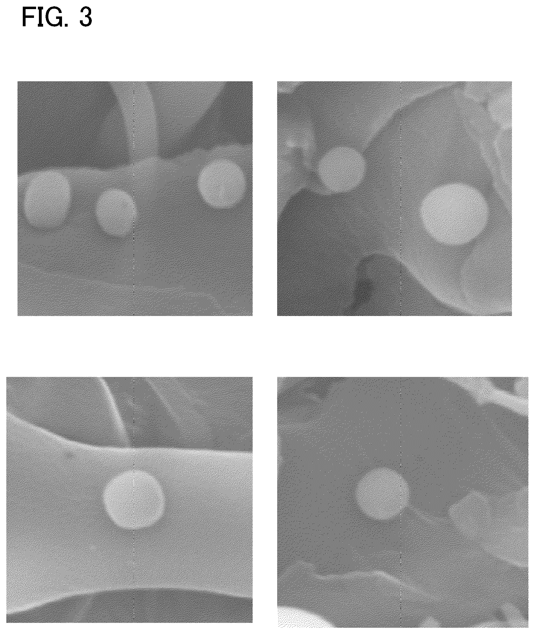

Fig. 3 is a photograph of MVs derived from AI in the upper part, and MVs derived from AM in the lower part. In general, the average particle size of MV is around 100 nm.

The anti-obesity agent of the present invention includes an Alistipes bacteria as the active ingredient, and when used as the anti-obesity agent, the bacteria may be in the form of either a killed bacteria such as a heated killed bacteria (HKB) or membrane vesicles (MVs), but the form of the membrane vesicle (MV) is more preferable from the viewpoint of migration to tissues and the like. Briefly describing the preparation method, the anti-obesity agent can be prepared by first passing through a step a of culturing Alistipes bacteria, and then filtering and centrifuging the culture solution cultured in the step a to obtain MVs (step b), or obtaining bacterial cells from the culture solution cultured in the step a, and heat-treating the bacterial cells to obtain heated killed bacteria (step c). From the viewpoint of tissue migration and the like, a method in which step a and step b are combined is preferable. Details are described in the following examples.

### Examples

The present invention is specifically described based on the following examples, but the present invention is not limited to these examples.

### (Example 1)

### <Culture of enterobacteria>

Akkermansia muciniphila (YIT 11774, referred to as "AM11774") and Alistipes indistinctus (YIT 12060, referred to as "AI12060") that had been cryopreserved were used, and were passaged at 37°C under anaerobic conditions with GAM (containing 1% glucose) medium and subjected to static culture. Lacticaseibacillus paracasei YIT 9037 (referred to as "LC9037") that had been cryopreserved was used, and was passaged at 37°C under anaerobic conditions with MRS (containing 1% lactose) medium and subjected to static culture.

Alistipes finegoldii (YIT 12685, referred to as "AF12685") appearing in Example 9 and subsequent Examples was also cultured in the same manner as in Example 1.

### (Example 2)

### <Cell>

A cryopreserved mouse fibroblast cell line 3T3-L1 was used. Note that 3T3-L1 is widely used as a cell model system of lipid metabolism because 3T3-L1 has a characteristic of accumulating lipid droplets in cells by being induced to differentiate into adipocytes. 3T3-L1 was passaged at 37°C in 5%CO2 using a DMEM medium (10% FBS/DMEM) containing 10% FBS and 100 µg/mL streptomycin.

### (Example 3)

### <Isolation of MV and production of heated killed bacteria>

MVs of AM11774, AI12060, and LC9037 were isolated from the culture supernatant 48 hours after passaging. Each cell culture solution was centrifuged (8,000 ×g, 20 min, 4°C) to remove bacterial cells, and a culture supernatant was collected.

The collected culture supernatant was passed through a PES syringe filter (Millipore) having a pore size of 0.45 µm, and then the culture supernatant containing MVs was concentrated by ultrafiltration (Millipore) with a molecular weight cutoff of 100 kDa. The concentrated culture supernatant was centrifuged (200,000 ×g, 2 h, 4°C) to pellet the MVs and suspended in a solvent suitable for subsequent measurements.

The MVs of AF12685 appearing in Example 9 and subsequent Examples are also produced by the same method as in Example 3.

The bacterial cells removed from the culture solution by centrifugation were suspended in MilliQ water and centrifuged (10,000 ×g, 10 min, 4°C) to wash the bacterial cells. This operation was repeated 3 times, and then a tube containing bacterial cells suspended in MilliQ water was placed in boiled hot water at 100°C for 10 minutes and heated. The heated bacterial cells were freeze-dried to obtain a powder, thereby producing heated killed bacteria (HKB).

The HKB of the AF12685 appearing in Example 9 and subsequent Examples is also produced by the same method as in Example 3.

### (Example 4)

### <Examination of action of inhibiting differentiation induction of adipocyte>

In a 24 well plate, 3T3-L1 was seeded at a predetermined number of cells (1.0 × 10⁵cells/well), and cultured at 37°C, 5% CO₂ for a total of 6 days until 3 days after confluence.

Thereafter, 10% FBS/DMEM in which Dexamethasone, 3-isobutyl-1-methylxanthine, and Insulin, which are differentiation inducing agents of AdipoInducer reagent kit (TAKARA), were added so that their respective final concentrations were 2.5 µM, 0.5 mM, and 10 µg/mL and HKB or MVs of each bacterium was treated at the same time, and the cells were cultured for 2 days.

After 2 days, the medium was replaced with 10 µg/mL Insulin-containing 10% FBS/DMEM containing HKB or MV, and cultivation was continued for another 2 days. Thereafter, the medium was replaced with a normal 10% FBS/DMEM medium, and 3 days later, the culture supernatant and the cells were used to analyze the amount of oil droplets in the cells, the expression levels of proteins and genes, and the production amount of adiponectin.

The test of the action of inhibiting differentiation induction of adipocytes by AF (and AI) appearing in Example 9 was also performed by the same method as in Example 4. Examples 10 and 11 partially overlap the procedure of Example 4. Details is described in each description of Examples 10 and 11.

### (Example 5)

### <Measurement of intracellular oil droplet amount by Oil-red O>

The culture supernatant of 3T3-L1 cultured in a 24 well plate was removed, washed once with PBS, then 10% neutral buffered formalin was added, and the mixture was allowed to stand at 4°C overnight to fix the cells.

Thereafter, the plate was washed with MilliQ water three times, an Oil-red O stain solution (Cosmo Bio) of a lipid assay kit was added thereto, and the plate was stained at room temperature for 1 hour. After staining, the washing was repeated several times with MilliQ water until the color of the Oil-red O stain disappeared from the washing solution. After washing, the cells were dried at 37°C, and cell stained images were photographed with a microscope.

The extract of the lipid assay kit (Cosmo Bio) was added to the stained 3T3-L1 and left for 30 minutes to extract Oil-red O. After extraction, the absorbance (505 nm) of the extract was measured with a plate reader to quantify the amount of intracellular oil droplets of 3T3-L1, and used as an index of the degree of differentiation into adipocytes.

The measurement of the intracellular oil droplet amount of AF (and AI) appearing in Example 9 and subsequent Examples by Oil-red O was also performed by the same method as in Example 5.

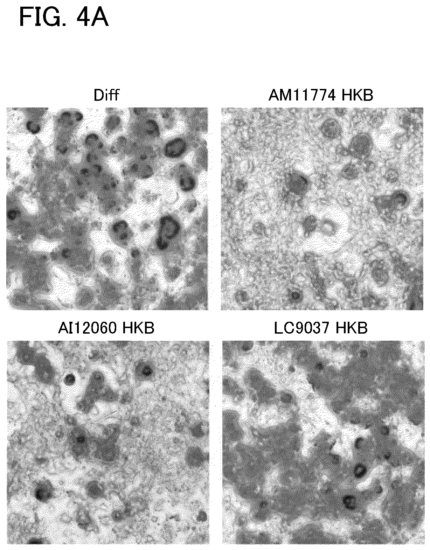

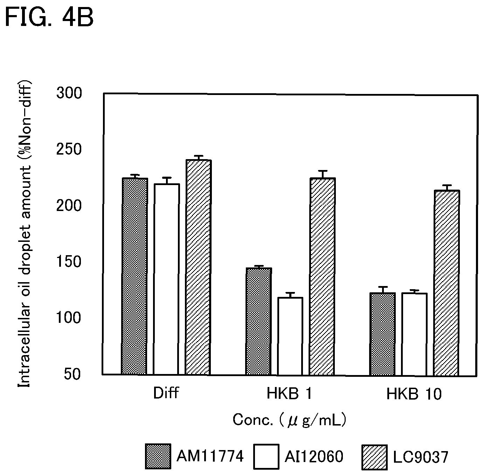

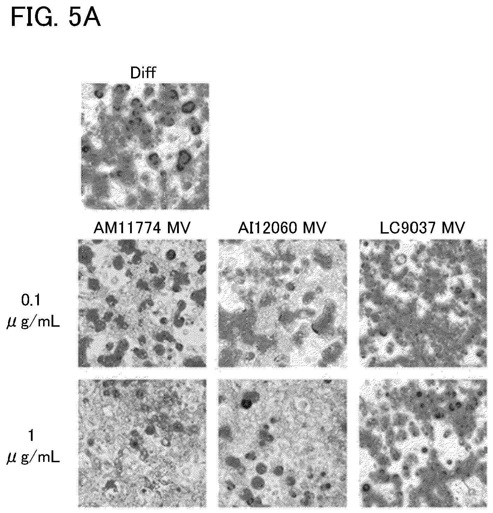

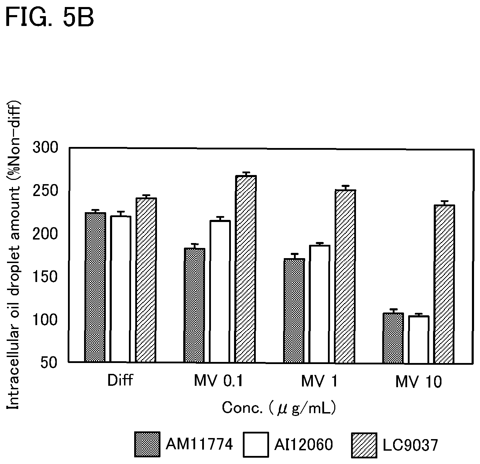

The results of Example 5 are shown in Figs. 4A, 4B, 5A, and 5B. These results examined whether AM and AI could suppress differentiation of adipocytes.

In the test of Figs. 4A and 4B, 1 µg/mL of HKB of each bacterium was added at the same time as the differentiation inducing agent was added to 3T3-L1, and after differentiation induction for 2 days, the cells were cultured for 5 days, and 3T3-L1 was stained with Oil-red O. Fig. 4A is a stained image (photograph) at that time.

In Fig. 4A, "Diff", "AM11774 HKB", "AI12060 HKB", and "LC9037 HKB" represent stained images of 3T3-L1 cultured by adding a control containing only a differentiation inducing agent, HKB of AM11774, HKB of AI12060, and HKB of LC9037, respectively.

From Fig. 4A, while "LC9037 HKB" had the same degree of staining as "Diff", "AM11774 HKB" and "AI12060 HKB" had a clearly lower degree of staining than "Diff".

Fig. 4B is a bar graph in which Oil-red O is extracted from stained 3T3-L1, and the amount of lipid oil droplets is quantified by absorbance. The vertical axis represents the absorbance in percentage, and the amount of lipid oil droplets of 3T3-L1 is quantified under each addition condition when the precursor cell (Non-diff) of undifferentiated 3T3-L1 is 100%. The horizontal axis represents a cell group added at each concentration of control (Diff) containing only a differentiation inducing agent, HKB 1 µg/mL (HKB1), and HKB 10 µg/mL (HKB10) in the order of left, center, and right.

From Fig. 4B, it was found that when HKB of "LC9037" was added, the accumulation of the amount of lipid oil droplets was not different from that in the control (Diff) using only the differentiation inducing agent, while "AM11774" and "AI12060" had a strong suppressing effect on the accumulation of the amount of lipid oil droplets at an HKB concentration of 1 µg/mL.

In the test of Fig. 5A and Fig. 5B, a differentiation inducing agent was added to 3T3-L1, and at the same time, MV of each bacterium was added at 0.1 µg/mL or 1 µg/mL, and after differentiation induction for 2 days, the cells were cultured for 5 days, and 3T3-L1 was stained with Oil-red O. Fig. 5A is a stained image (photograph) at that time.

In Fig. 5A, "Diff", "AM11774 MV", "AI12060 MV", and "LC9037 MV" indicate the stained images of 3T3-L1 cultured with the addition of the differentiation inducing agent only control, the MV of AM11774, the MV of AI12060, and the MV of LC9037, respectively.

From Fig. 5A, "LC9037 MV" was no different staining degree than "Diff" at any concentration, while "AM11774 MV" and "AI12060 MV" were clearly lower in staining degree compared to them. The degree of staining of "AM11774 MV" and "AI12060 MV" reduced in a concentration-dependent manner.

Fig. 5B is a bar graph in which Oil-red O is extracted from stained 3T3-L1, and the amount of lipid oil droplets is quantified by absorbance. The vertical axis represents the absorbance in percentage, and the amount of lipid oil droplets of 3T3-L1 is quantified under each addition condition when the precursor cell (Non-diff) of undifferentiated 3T3-L1 is 100%. The horizontal axis represents a cell group added at each concentration of control (Diff) containing only a differentiation inducing agent, MV 0.1 µg/mL (MV0.1), MV 1 µg/mL (MV1), and MV 10 µg/mL (MV10) in the order of left, center left, center right, and right.

From Fig. 5B, it was found that when MV of "LC9037" was added, the accumulation of the amount of lipid oil droplets was not different from that in the control (Diff) using only the differentiation inducing agent, while "AM11774" and "AI12060" had a strong suppressing effect on the accumulation of the amount of lipid oil droplets at an MV concentration of 0.1 µg/mL. In addition, the suppressing effect increased in a concentration-dependent manner.

From the above results, it was confirmed that HKB and MVs of AM and AI had a strong suppressing effect on the accumulation of the amount of lipid oil droplets in adipocytes, and the suppressing effect increased in a concentration-dependent manner.

### (Example 6)

### <Protein expression analysis by Western blot>

A cell solubilizing solution (10 mM Tris-HCl, pH7.4, 0.1% NP-40, 0.1% sodium deoxycholate, 0.1% SDS, 0.15M NaCl, 1 mM EDTA, 10 µg/mL aprotinin) was added to a 24 well plate (Corning) of 3T3-L1 cultured according to Example 4 to lyse the cells. This lysate was subjected to electrophoresis using a 10% acrylamide gel, and the proteins in the gel were transferred to an Immobilon PVDF membrane using a semi-dry transfer apparatus.

After transfer, the membrane was blocked and immersed overnight in a primary antibody solution of an anti-PPAR-γ rabbit monoclonal antibody (Cell signaling, #2443S), an anti-CEBP rabbit polyclonal antibody (Cell signaling, #2295S), and an anti-GAPDH mouse monoclonal antibody (Thermo, #AM4300).

Furthermore, after immersing for 1 hour in a secondary antibody solution of peroxidase-labeled anti-mouse IgG antibody or anti-rabbit IgG antibody (GE Healthcare), the target protein on the membrane was detected using ECL Prime Western Blotting Detection System or ECL Select Western Blotting Detection System (Cytiva).

The method of protein expression analysis by Western blot appearing in Example 9 is also the same as that in Example 6. In Example 9, β-actin is used as an internal standard instead of GAPDH.

### (Example 7)

### <Gene expression analysis by real-time PCR>

3T3-L1 cultured in a 24 well plate (Corning) according to Example 4 was recovered, and total RNA was purified by a predetermined method using illustra RNAspin kit (Cytiva). Purified RNA was reverse transcribed by GoScript (trademark) Reverse Transcriptase (Promega) and real-time PCR was performed using GoTaq (registered trademark) qPCR Master Mix (Promega) and QuantStudio (trademark) 12K Flex Real-Time PCR System (Applied Biosystems) according to the attached operating procedures.

As primers for the PPAR-γ gene, PPAR-γ_Fw(5'-GGGCGATCTTGACAGGAAAG-3') and PPAR-γ_Rv(5'-CCCATCATTAAGGAATTCATGTCAT-3') were designed from known mRNA base sequences using Primer Express Software Version 3.0 (Applied Biosystems).

The method of gene expression analysis by real-time PCR appearing in Example 9 is also the same as that in Example 7.

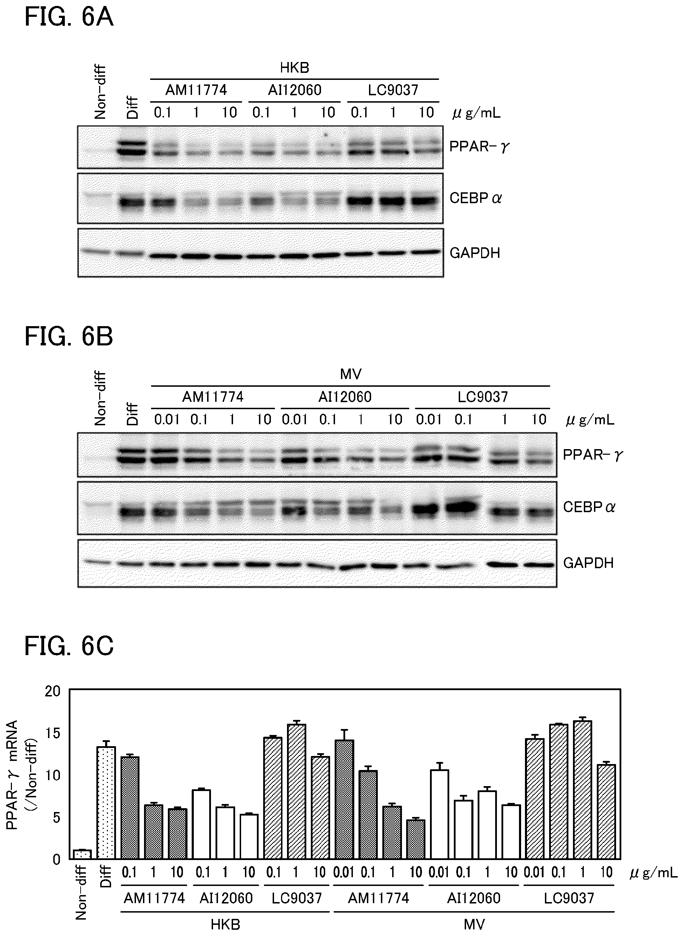

The results of Examples 6 and 7 are shown in Figs. 6(A) to 6(C). Figs. 6(A) to 6(C) show that whether HKB or MV of AM and AI has an effect of suppressing differentiation of 3T3-L1 of adipocytes is examined through the protein expression level (Figs. 6(A) and 6(B)) or mRNA expression level (Fig. 6(C)) which is related to differentiation of adipocytes. In each figure, "Non-diff" represents undifferentiated 3T3-L1, and "Diff" represents 3T3-L1 subjected only to differentiation induction treatment. "GAPDH" is an internal standard.

In Fig. 6(A), the expression levels of PPAR-γ and CEBPα proteins 3T3-L1 after adding HKB of AM11774, AI12060 and LC9037 at the same time as the addition of the differentiation inducing agent, and culturing for 5 days after differentiation induction for 2 days were confirmed by Western blot.

PPAR-γ is a protein belonging to the nuclear receptor superfamily and is a master regulator expressed when differentiated into adipocytes. CEBPα protein is its cofactor. By measuring the expression levels of respective proteins of PPAR-γ and CEBPα, the degree of differentiation of 3T3-L1 can be confirmed.

The expression levels of the respective proteins of PPAR-γ and CEBPα were confirmed by Western blot, and when HKB of AI12060 and AM11774 was added, the expression of the respective proteins of PPAR-γ and CEBPα was suppressed around the addition amount of 0.1 µg/mL.

In Fig. 6(B), the expression levels of PPAR-γ and CEBPα proteins of 3T3-L1 after adding MVs of AM11774, AI12060, and LC9037 simultaneously with the addition of the differentiation inducing agent, and culturing for 5 days after differentiation induction for 2 days were confirmed by Western blot. As a result, as in the case of HKB in Fig. 6(A), the expression of each protein of PPAR-γ and CEBPα was suppressed from around the addition amount of 0.1 µg/mL.

Fig. 6(C) shows the expression level of PPAR-γ mRNA in Figs. 6(A) and 6(B). The mRNA amount of PPAR-γ in each cell is corrected by the mRNA expression level of GAPDH as an internal standard. As a result, it was confirmed that the expression of mRNA was suppressed in parallel with the protein expression levels in Figs. 6(A) and 6(B).

From the above results, it was confirmed that HKB and MVs of AM and AI have an effect of suppressing differentiation of 3T3-L1 of adipocytes in the level of protein expression and the level of mRNA expression. Therefore, HKB and MVs of AM and AI could be regarded as the active ingredients of the anti-obesity agent, particularly a differentiation suppressant of adipocytes.

### (Example 8)

### <Measurement of adiponectin amount in culture supernatant>

Detection of adiponectin in the culture supernatant was performed by an ELISA method using Human Adiponectin/Acrp30 DuoSet (R & Dsystems). The capture antibody was diluted with PBS to a predetermined concentration, added to a 96 well plate for ELISA in 100 µL portions, and incubated at 4°C overnight. Each well was washed with PBS (washing solution) containing 0.05% (v/v) Tween 20, then 100 µL each of PBS containing 1% (w/v) BSA (BSA/PBS) was added, and blocking was performed at 37°C for 90 minutes.

After blocking, 100 µL each of a culture supernatant diluted 1000 times with 1% (w/v) BSA/PBS was added thereto, and the mixture was reacted overnight at 4°C. After the reaction, washing operation was performed 3 times, 100 µL each of Detection antibody diluted to a predetermined concentration with 1% (w/v) BSA/PBS was added, and the mixture was reacted at room temperature for 120 minutes. Then, 100 µL each of Streptavidin-HRP diluted to a predetermined concentration with 1% (w/v) BSA/PBS was added, and the mixture was reacted at dark room temperature for 20 minutes.

Thereafter, the mixture was washed 3 times with a washing solution, 50 µL each of 1-Step (trademark) Ultra TMB-ELISA Substrate Solution (Thermo Fisher Scientific) was added thereto, and the mixture was reacted at room temperature for 20 minutes. After confirming the color development, 50 µL each of 2M H₂SO₄ was added to stop the reaction, and the absorbances at 450 nm and 595 nm were measured with a microplate reader.

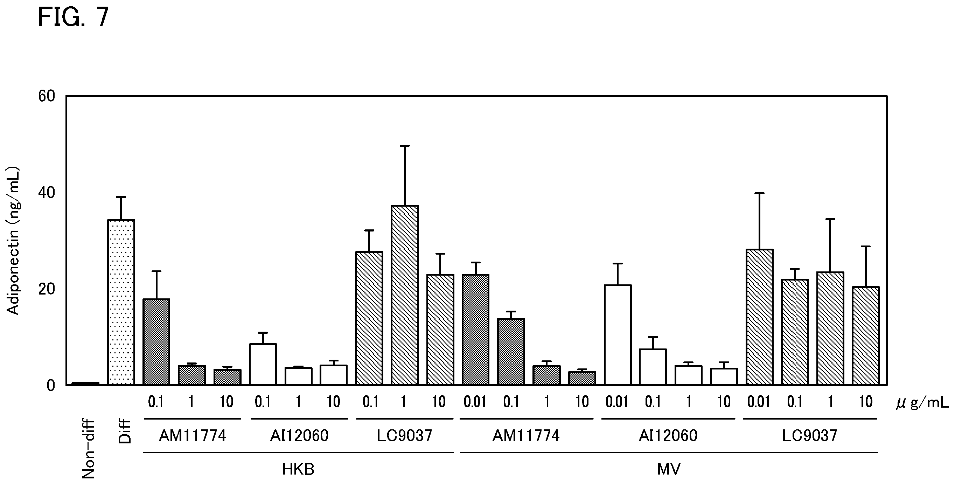

The results of Example 8 are shown in Fig. 7. Fig. 7 shows the results of confirming whether HKB or MVs of AM and AI has an effect of suppressing differentiation of 3T3-L1 of adipocytes through the amount of secretion of adiponectin. Adiponectin is a type of adipocytokine, and is a physiologically active protein secreted from differentiated adipocytes.

Fig. 7 is a bar graph showing the results of culturing 3T3-L1 after addition of HKB or MVs, collecting the culture supernatant, and measuring the amount of adiponectin by ELISA. "Non-diff" refers to undifferentiated 3T3L-1, and "Diff" refers to 3T3-L1 subjected only to differentiation induction treatment. The vertical axis represents the amount of adiponectin (ng/mL), and the horizontal axis represents 3T3-L1 to which MVs or HKB of each bacterium is added.

From Fig. 7, similarly to the expression level of PPAR-γ, when HKB and MVs of AI12060 and AM11774 were added, the secretion amount of adiponectin into the culture supernatant by 3T3-L1 was also small. From the above, it was further confirmed that HKB and MVs of AI12060 and AM11774 suppressed the differentiation of 3T3-L1 into adipocytes.

From the results of Examples 1 to 8, it became clear that HKB and MVs of AM and AI showed an effect of suppressing differentiation into adipocytes. Therefore, in Example 9 and subsequent examples, focusing on HKB and MVs of Alistipes bacteria, in particular, and Alistipes indistinctus and Alistipes finegoldii, the action of suppressing differentiation, the action of suppressing lipid accumulation, and the action of reducing accumulated lipid were examined.

### (Example 9)

### <Examination of action of suppressing differentiation of AI and AF>

From the results of Examples 1 to 8, it became clear that HKB and MVs of AI each have an anti-obesity effect, particularly an action of suppressing differentiation of adipocytes. Therefore, whether AF, which is the same Alistipes bacteria, has a similar action was examined together with AI using a similar method. The analysis method is the same as the analysis method of Examples 1 to 7 described above.

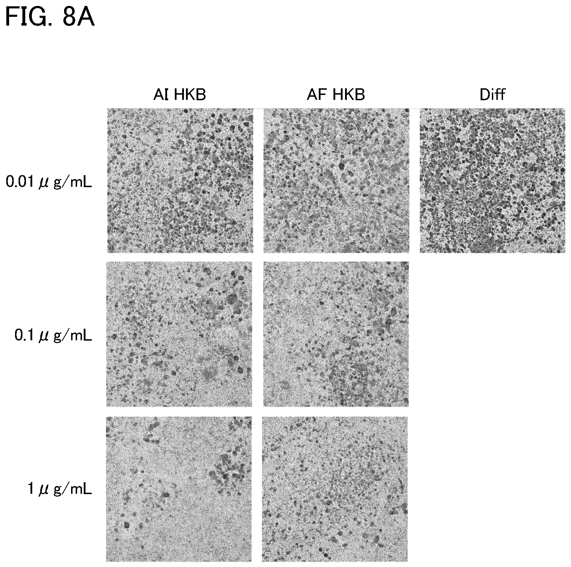

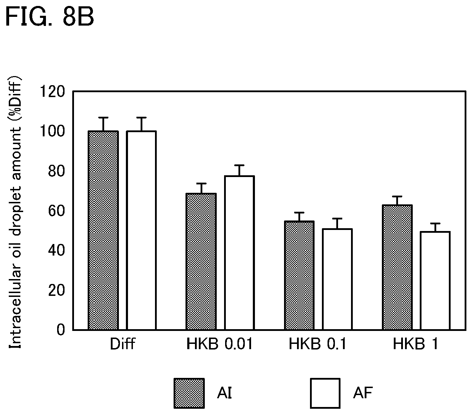

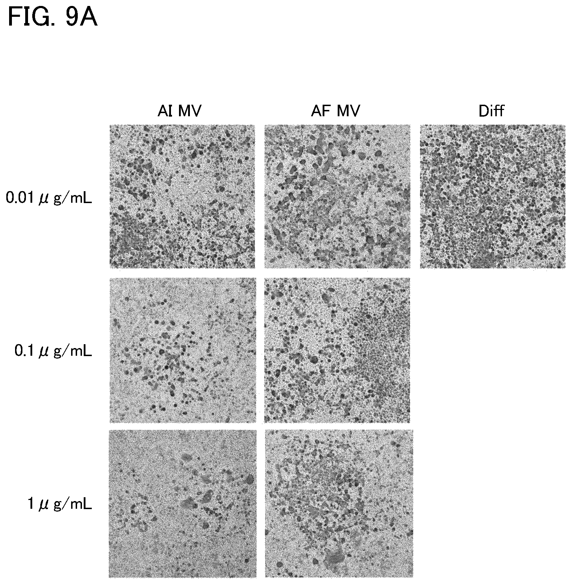

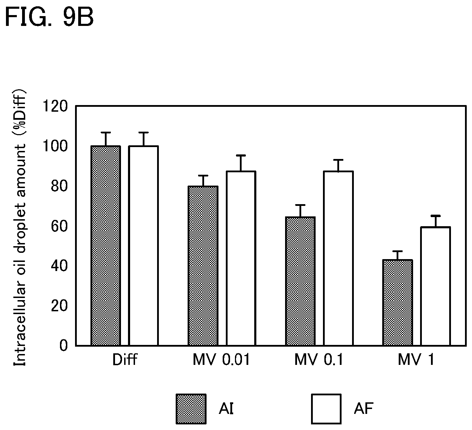

These results are shown in Figs. 8 to 10. First, in Figs. 8A, 8B, 9A, and 9B, it was examined whether or not the enterobacteria of AF and AI have a suppressing effect on the accumulation of the amount of lipid oil droplets, which is a characteristic of differentiated adipocytes.

Figs. 8A and 8B show the results of Oil-red O staining of 3T3-L1 to which HKB of AF and AI were added simultaneously with the addition of the differentiation inducing agent. Fig. 8A is a stained image by Oil-red O staining of 3T3-L1 obtained by adding HKB of each bacterium in the range of 0.01 µg/mL to 1 µg/mL, inducing differentiation for 2 days, and culturing for 5 days. "Diff" is a control of only the differentiation inducing agent.

It was confirmed that the degree of staining of AF and AI reduced in a concentration-dependent manner as compared with the control using only the differentiation inducing agent.

Fig. 8B is a bar graph in which Oil-red O is extracted from stained 3T3-L1, and the amount of lipid oil droplets is quantified by absorbance. The vertical axis represents the absorbance in percentage, and the amount of lipid oil droplets of 3T3-L1 is quantified under each addition condition when the control (Diff) of only the differentiation inducing agent is 100%. The horizontal axis represents a cell group added at each concentration of control (Diff) containing only a differentiation inducing agent, HKB 0.01 µg/mL (HKB0.01), HKB 0.1 µg/mL (HKB0.1), and HKB 1 µg/mL (HKB1) in the order of left, center left, center right, and right.

From Fig. 8B, it was confirmed that AF and AI had a strong suppressing effect on the accumulation of the amount of lipid oil droplets at a HKB concentration of 0.01 µg/mL, and the effect increased in a concentration-dependent manner, as compared with the control using only the differentiation inducing agent.

Figs. 9A and 9B are the results of Oil-red O staining of 3T3-L1 to which of MVs of AF and AI were added simultaneously with the addition of a differentiation inducing agent. Fig. 9A is a stained image by Oil-red O staining of 3T3-L1 obtained by adding MV of each bacterium in the range of 0.01 µg/mL to 1 µg/mL, inducing differentiation for 2 days, and culturing for 5 days. "Diff" is a control of only the differentiation inducing agent.

It was confirmed that the degree of staining of AF and AI reduced in a concentration-dependent manner as compared with the control using only the differentiation inducing agent.

Fig. 9B is a bar graph in which Oil-red O is extracted from stained 3T3-L1, and the amount of lipid oil droplets is quantified by absorbance. The vertical axis represents the absorbance in percentage, and the amount of lipid oil droplets of 3T3-L1 is quantified under each addition condition when the control (Diff) of only the differentiation inducing agent is 100%. The horizontal axis represents a cell group added at each concentration of control (Diff) containing only a differentiation inducing agent, MV 0.01 µg/mL (MV0.01), MV 0.1 µg/mL (MV0.1), and MV 1 µg/mL (MV1) in the order of left, center left, center right, and right.

From Fig. 9B, it was confirmed that AF and AI had a strong suppressing effect on the accumulation of the amount of lipid oil droplets at a MV concentration of 0.01 µg/mL, and the effect increased in a concentration-dependent manner, as compared with the control using only the differentiation inducing agent.

From the above results, it was confirmed that HKB and MVs of AF and AI had a strong suppressing effect on the accumulation of the amount of lipid oil droplets in adipocytes, and the suppressing effect increased in a concentration-dependent manner.

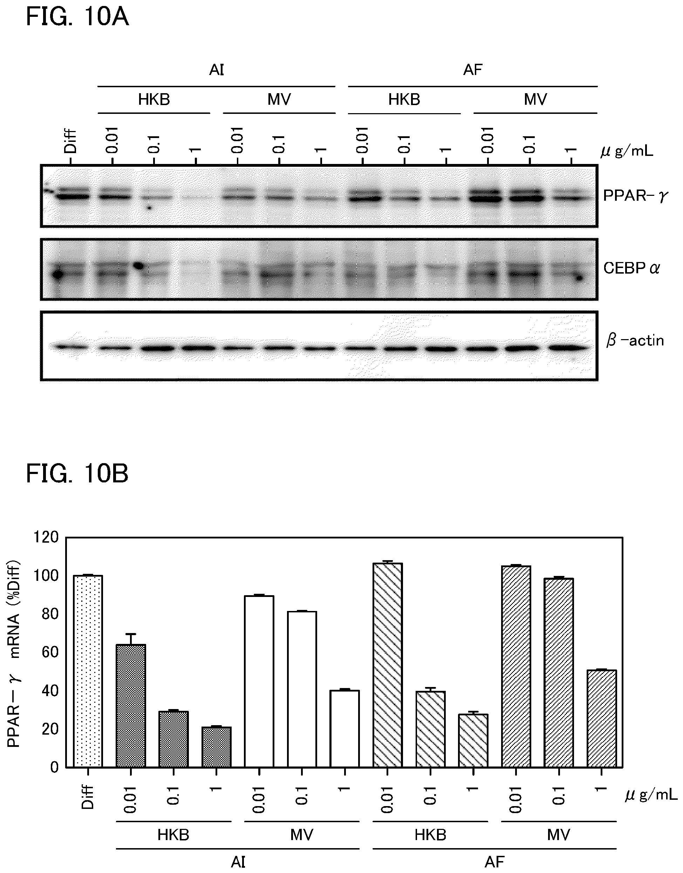

Next, Fig. 10 shows whether or not HKB or MVs of AF and AI has an effect of suppressing differentiation of 3T3-L1 of adipocytes, which was confirmed through the protein expression level (Fig. 10 (A)) or mRNA expression level (Fig. 10 (B)) related to differentiation of adipocytes.

Incidentally, the method of protein expression analysis appearing in Example 9 is the same as that in Example 6, but β-actin is used instead of GAPDH as an internal standard.

In Fig. 10 (A), the expression levels of PPAR-γ and CEBPα proteins of 3T3-L1 after adding AF, HKB or MVs of AF and AI at the same time as the addition of the differentiation inducing agent, and culturing for 5 days after inducing differentiation for 2 days were confirmed by Western blot.

It was confirmed that the addition of HKB or MVs of AF and AI suppressed the expression of proteins of PPAR-γ and CEBPα proteins in a concentration-dependent manner.

Fig. 10 (B) shows the expression level of mRNA of PPAR-γ in Fig. 10 (A). The mRNA amount of PPAR-γ in each cell is corrected by the mRNA expression level of β-actin as an internal standard. As a result, it was confirmed that the expression of mRNA was suppressed in parallel with the protein expression level in Fig. 10 (A).

From the above results, it was confirmed that HKB and MVs of AF and AI have an effect of suppressing differentiation of 3T3-L1 of adipocytes in the level of protein expression and the level of mRNA expression. Therefore, similarly to AI, HKB and MVs of AF, which are the same Alistipes bacteria, could also be regarded as active ingredients of the differentiation suppressant for adipocytes.

### (Example 10)

### <Study on action of suppressing lipid accumulation by AI and AF>

Studies of Examples 1 to 9 have revealed that Alistipes bacteria, for example, AI and AF, are useful as the anti-obesity agent, particularly as the differentiation suppressant for adipocytes.

That is, the present agent has an action of suppressing the process of differentiation from the precursor cells 102 to the small adipocytes 103 in Fig. 1. Therefore, in Example 10, it was examined whether Alistipes bacteria have an action of suppressing a process in which small adipocytes 103 excessively accumulate lipid droplets to form enlarged adipocytes 104, that is, an action of suppressing lipid accumulation.

The analysis method is the same as the analysis methods of Examples 1 to 3 and 5 described so far. However, in order to examine whether AF and AI have an action of suppressing lipid accumulation, only the method of Example 4 was partially changed (Hereinafter, referred to as "Example 4A".). Details are as follows.

### <<Example 4A>>

In a 24 well plate, 3T3-L1 was seeded at a predetermined number of cells (1.0 × 10⁵cells/well), and cultured at 37°C, 5% CO₂ for a total of 6 days until 3 days after confluence.

Thereafter, 10% FBS/DMEM in which Dexamethasone, 3-isobutyl-1-methylxanthine, and Insulin, which are differentiation inducing agents of AdipoInducer reagent kit (TAKARA), were added so that their respective final concentrations were 2.5 µM, 0.5 mM, and 10 µg/mL, and the cells were cultured for 3 days.

After 3 days, the medium was replaced with 10% FBS/DMEM containing 10 µg/mL of Insulin, and at the same time, 1 µg/mL of MV of each bacteria was treated to prepare wells and untreated wells, and culture was further performed for 21 days. Medium exchange was performed every 2 to 3 days in an Insulin-containing 10% FBS/DMEM containing 500 µL of each bacteria MV. Thereafter, the amount of oil droplets in the cells was examined using Oil-red O.

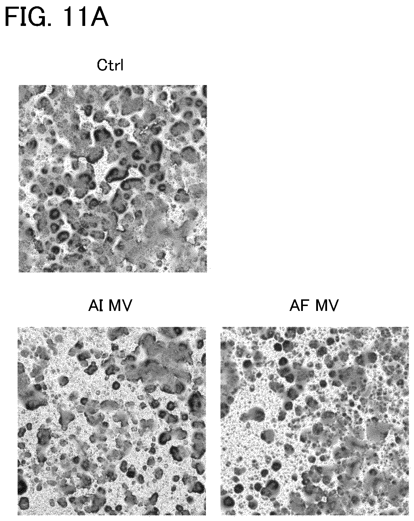

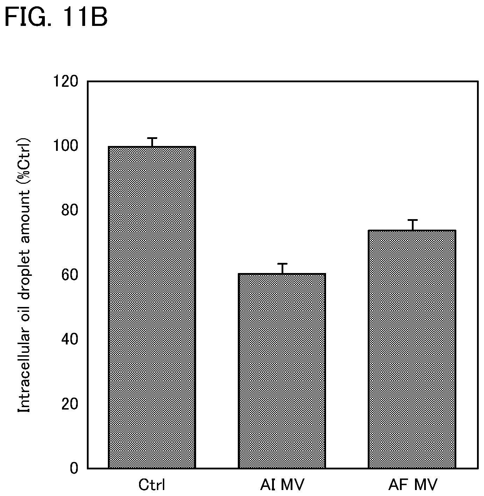

The results are shown in Figs. 11A and 11B. Figs. 11A and 11B show the results of Oil-red O staining of 3T3-L1 to which MVs of AF and AI were added 3 days after culture from the addition of the differentiation inducing agent. Fig. 11A is a stained image by Oil-red O staining of 3T3-L1 after adding 1 µg/mL of MVs of AF and AI and culturing for 21 days. "Ctrl" is a control of only the differentiation inducing agent. AF and AI showed a clearly reduced degree of staining compared to the control.

Fig. 11B is a bar graph in which Oil-red O is extracted from stained 3T3-L1, and the amount of lipid oil droplets is quantified by absorbance. The vertical axis represents the absorbance in percentage, and the amount of lipid oil droplets of 3T3-L1 is quantified under each addition condition when the control (Ctrl) of only the differentiation inducing agent is 100%. The horizontal axis represents a cell group to which control, AI MV, and AF MV are added in the order of left, center, and right.

From Fig. 11B, it was found that AF and AI have a strong suppressing effect on the accumulation of the amount of lipid oil droplets as compared with the control.

From the above study, it has become clear that Alistipes bacteria, for example, AI and AF, are also useful as the anti-obesity agent, particularly, the lipid accumulation suppressant of adipocytes. That is, the present agent has an action of suppressing a process in which the small adipocytes 103 excessively accumulate lipid droplets to form the enlarged adipocytes 104.

### (Example 11)

### <Study on action of reducing accumulated lipid by AI and AF>

Studies up to Examples 1 to 10 have revealed that Alistipes bacteria, such as AI and AF, are useful as the anti-obesity agent, particularly as the differentiation suppressant or the lipid accumulation suppressant for adipocytes.

Therefore, in Example 11, it was examined whether Alistipes bacteria have an action of reducing lipid droplets already accumulated in the enlarged adipocytes 104.

The analysis method is the same as the analysis methods of Examples 1 to 3 and 5 described so far. However, in order to examine whether AF and AI have an action of reducing accumulated lipid, only a part of the method of Example 4 was changed (Hereinafter, referred to as "Example 4B".). Details are as follows.

### <<Example 4B>>

In a 24 well plate, 3T3-L1 was seeded at a predetermined number of cells (1.0 × 10⁵cells/well, and cultured at 37°C, 5% CO₂ for a total of 6 days until 3 days after confluence.

Thereafter, 10% FBS/DMEM in which Dexamethasone, 3-isobutyl-1-methylxanthine, and Insulin, which are differentiation inducing agents of AdipoInducer reagent kit (TAKARA), were added so that their respective final concentrations were 2.5 µM, 0.5 mM, and 10 µg/mL, and the cells were cultured for 2 days.

After 2 days, the medium was replaced with 10% FBS/DMEM containing 10 µg/mL of Insulin, and further cultured for 21 days to produce enlarged adipocytes. Medium exchange was performed every 2 to 3 days in 10% FBS/DMEM containing 500 µL of Insulin.

Thereafter, wells treated with 1 µg/mL MV of each bacterium and untreated wells were prepared, and continuously cultured for 10 days while replacing the medium with 10% FBS/DMEM containing MV of each bacterium every 2 to 3 days. Using the cells, the amount of oil droplets in the cells was examined using Oil-red O.

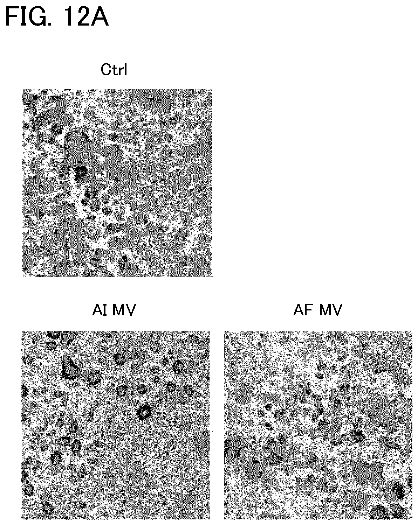

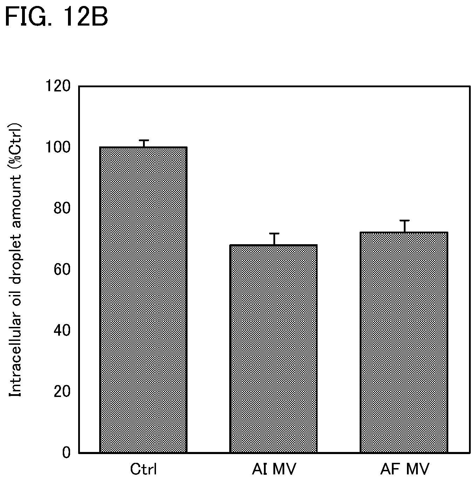

The results are shown in Figs. 12A and 12B. Figs. 12A and 12B show the results of Oil-red O staining of 3T3-L1 to which MVs of AF and AI were added after producing enlarged adipocytes by adding a differentiation inducing agent. Fig. 12A is a stained image by Oil-red O staining of 3T3-L1 after adding 1 µg/mL of MVs of AF and AI and culturing for 10 days. "Ctrl" is a control of only the differentiation inducing agent. AF and AI showed a clearly reduced degree of staining compared to the control.

Fig. 12B is a bar graph in which Oil-red O is extracted from stained 3T3-L1, and the amount of lipid oil droplets is quantified by absorbance. The vertical axis represents the absorbance in percentage, and the amount of lipid oil droplets of 3T3-L1 is quantified under each addition condition when the control (Ctrl) of only the differentiation inducing agent is 100%. The horizontal axis represents a cell group added at each concentration of control (Ctrl) containing only a differentiation inducing agent, MV of AI, and MV of AF, respectively in the order of left, center, and right.

From Fig. 12B, it was found that AF and AI have a strong reducing effect on the accumulation of the amount of lipid oil droplets as compared with the control.

From the above study, it has become clear that Alistipes bacteria, for example, AI and AF, are also useful as the anti-obesity agent, in particular, as an agent for reducing accumulated lipid in adipocytes. That is, the present agent has an action of reducing lipid droplets already accumulated by the enlarged adipocytes 104.

From the results of Examples 1 to 11 described above, it became clear that the anti-obesity agent of the present invention has an action of suppressing the process in which the precursor cells 102 are differentiated into the small adipocytes 103, suppressing the process in which the small adipocytes 103 excessively accumulate lipid droplets to form the enlarged adipocytes 104, and further reducing the lipid droplets already accumulated by the enlarged adipocytes 104.

Further, in Examples, the present inventors isolated heated killed bacteria (HKB) and membrane vesicles (MVs) from Alistipes bacteria and used them for each test. However, since Alistipes bacteria themselves generate the active ingredients, even when Alistipes bacteria themselves are contained in the anti-obesity agent, the anti-obesity agent effectively acts.

In consideration of the results of Examples and the mechanism of obesity described so far, the present invention effectively acts not only as the anti-obesity agent but also as a therapeutic agent for diabetes, a therapeutic agent for hyperlipidemia, a therapeutic agent for hypertension, a therapeutic agent for a lifestyle disease, or a prophylactic agent for these diseases.

In addition, it is desirable that the anti-obesity agent of the present invention ingests 1 × 10² or more and more preferably 1 × 10⁶ or more Alistipes bacteria as the active ingredient per day as a lower limit. In addition, it is desirable to ingest 1 × 10¹⁵ or less, more preferably 1 × 10¹¹ or less per day as an upper limit.

In addition, it is desirable that the anti-obesity agent of the present invention is continuously ingested over a long period of time until the obesity is ideally eliminated. In order to better exhibit the effect, as a lower limit, for example, it is preferable to continuously ingest for four weeks or more, and it is more preferable to continuously ingest for several months to several years or more.

### (Example 12)

### <Tissue migration test of MV using mouse>

In the studies so far, HKB and MVs showed the same degree of the effect of suppressing differentiation, the effect of suppressing lipid accumulation, and effect of reducing lipid droplets already accumulated in the enlarged adipocytes as compared with 3T3-L1. Furthermore, in order to examine the possibility that orally administered MV directly acts on adipocytes, tissue migration in oral administration to mice was examined.

### <<Oral administration to mouse>>

Six-week-old female C57BL/6JJcl mice (CLEA Japan) were purchased and used. After one week of conditioning, MVs of AI12060 and AM11774 were suspended in PBS at 1 mg/mL (in terms of protein amount), and 0.2 mL was orally administered so that the total amount of MVs was 200 µg. Feces were collected 1 hour, 6 hours, and 16 hours after administration, and were euthanized by total cardiac blood collection under anesthesia, and then brain, lung, spleen, pancreas, liver, large intestine, and adipose tissue were extracted and used for subsequent experiments.

Since the 16S rRNA contained in the MVs is rapidly degraded when the vesicles as the MVs are disintegrated and exposed to the outside, it can be confirmed whether the MVs transferred into each tissue maintain the shape by detecting the 16S rRNA of the bacterial cells. Therefore, qPCR was performed by the following method, and MV in the feces, blood, and each tissue (brain, lung, spleen, pancreas, liver, large intestine, adipose tissue ) after 1, 6, and 16 hours was measured.

### <<Extraction of total RNA>>

RNA extraction from MV or mouse tissues was performed using Illustra^{™} RNAspin (Cytiva) for measurement. Cell lysis buffer (Buffer RA1) attached to the kit was added, and MVs and tissue were completely lysed using BioMasher (Nippi). In the following operations, total RNA was extracted according to the attached procedure manual.

### <<Quantification of MVs by RT-qPCR using 16S rRNA>>

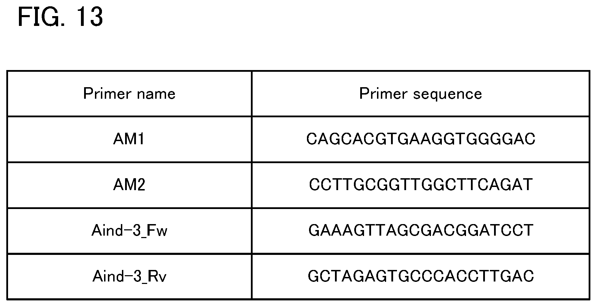

qPCR was performed using the GoTaq^{®} 1-Step RT-qPCR System (Promega) and QuantStudio^{™} 12K Flex Real-Time PCR System (Applied Biosystem) according to the attached operating procedures. A primer for detecting 16S rRNA of each bacterial cell is shown in Fig. 13.

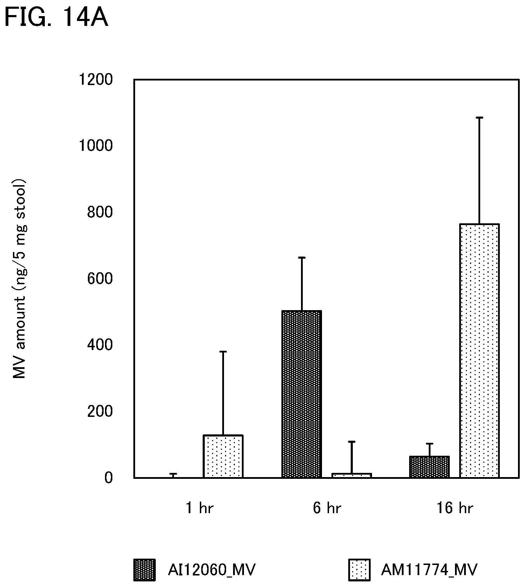

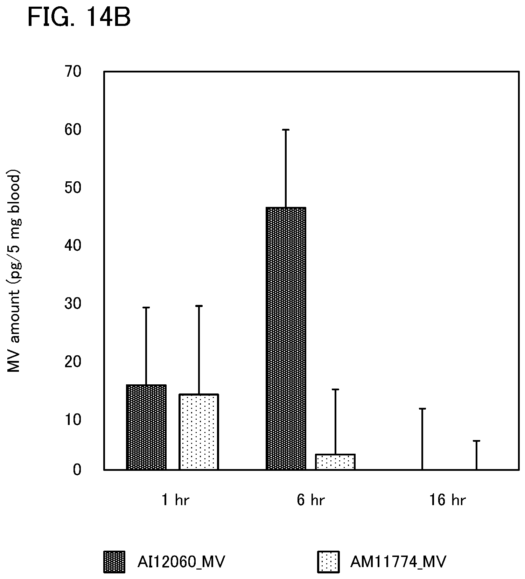

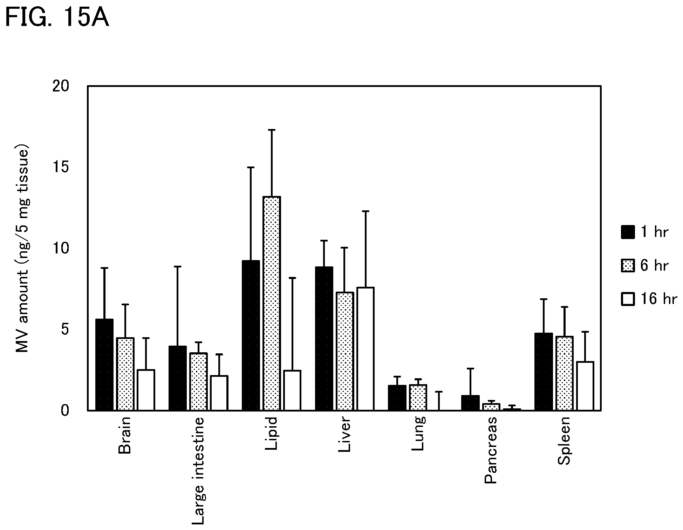

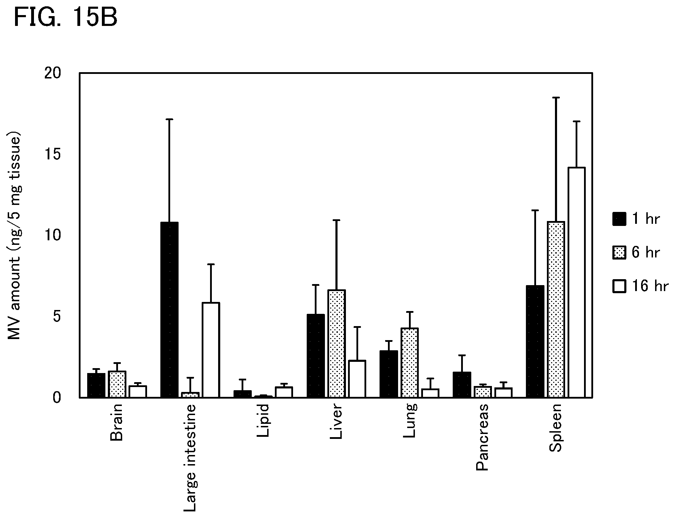

Fig. 14A shows the detection amounts of MVs in the feces per 5 mg at each time (1, 6, and 16 hours) after oral administration, and Fig. 14B shows the detection amounts of MVs in the whole blood at each time (1, 6, and 16 hours) after oral administration. Fig. 15A shows the detection amounts of MVs in each tissue per 5 mg at each time (1, 6, and 16 hours) after oral administration of MV of AI12060, and Fig. 15B shows the MV detection amount in each tissue per 5 mg at each time (1, 6, and 16 hours) after oral administration of MV of AM11774. Since the detection amounts of MVs in the tissue may have an error from the actual MVs amount due to the influence of RNA and the like derived from cells and tissues, total RNA was extracted under mixing of 5 mg of each tissue (brain, spleen, lung, liver, lipid, large intestine, pancreas) of the mouse and 5 µg of each MVs (AI12060, AM11774) and confirmed by qPCR, and a deviation value was obtained and corrected by the error.

From these results, it was found that the permeation of MVs through the intestinal tract, the transfer of MVs to the blood, and the transfer of MVs to each tissue were rapid. In addition, from Figs. 15A and B, it was confirmed that the MVs of AM11774 hardly showed migration to adipose tissue, whereas the MVs of AI12060 showed high migration to adipose tissue. As a result, it was revealed that MVs of AI12060 orally administered easily migrates to adipose tissue and directly acts on adipocytes, and thus exhibits an anti-obesity effect.

Therefore, based on the above results, it has become clear that the anti-obesity agent of the present invention is Alistipes bacteria, and in particular, it is more preferable to contain bacterial membrane vesicles (MVs) as the active ingredient.

The present application claims priority based on Japanese Patent Application No. 2022-136372 and Japanese Patent Application No. 2022-136373 filed on August 29, 2022, the entire contents of which are incorporated herein by reference.

### Reference Signs List

- 101: Mesoderm stem cell
- 102: Precursor cells
- 103: Small adipocytes
- 104: Enlarged adipocytes
- 201: Enterobacteria
- 202: MVs
- 203: Intestinal epithelium
- 204: Blood vessel
- 205: Dendritic cell
- 206: T cells

## Claims

1. An anti-obesity agent containing Alistipes bacteria or membrane vesicles (MVs) thereof as an active ingredient.

2. The anti-obesity agent according to claim 1, wherein the anti-obesity agent is an adipocyte differentiation suppressant, a lipid accumulation suppressant, or an agent for reducing accumulated lipid.

3. The anti-obesity agent of claim 1 or claim 2, wherein the bacteria is Alistipes indistinctus or Alistipes finegoldii.

4. A method for producing an anti-obesity agent containing Alistipes bacteria or membrane vesicles (MVs) thereof as an active ingredient, the method comprising: a step a of culturing Alistipes bacteria; and
a step b of acquiring the bacteria from the culture solution cultured in the step a.

5. The method for producing the anti-obesity agent according to claim 4, wherein the step b further includes a step of centrifuging and filtering the culture solution to obtain the MVs.
